# EUROPEAN PATENT APPLICATION

(11) **EP 3 381 497 A1**
(43) Date of publication of application: **03.10.2018**
(21) Application number: 18162486.7
(22) Date of filing: 19.03.2018
(51) Int. Cl.: A61M 25/01, A61B 1/005, A61B 1/008

(54) **OPERATION MECHANISM, ENDOSCOPE, AND MANUFACTURING METHOD**

(30) Priority: 29.03.2017 JP 2017065294
(71) Applicant: FUJIFILM Corporation, Tokyo 106-8620 (JP)
(72) Inventor: KATO, Kota, Ashigarakami-gun, Kanagawa 258-8538 (JP); FUJIWARA, Nobuhiko, Ashigarakami-gun, Kanagawa 258-8538 (JP); KARAKI, Hideyuki, Ashigarakami-gun, Kanagawa 258-8538 (JP)
(74) Representative: Jeffrey, Philip Michael

(57) **Abstract**

An object of the invention is to provide an operation mechanism, an endoscope, and a manufacturing method that can reliably fix an operation wire to a member to be operated. According to an operation mechanism of an aspect of the invention, since a sleeve member is caulked in a caulked shape where a twisted shape of a plurality of strands of an operation wire is maintained, the sleeve member is caulked while the strands are maintained in an original line contact state. Accordingly, since damages to the strands caused by a point contact state of the strands do not occur, the tensile strength and bending durability of the operation wire are improved and the operation wire can be reliably fixed to a member to be operated. Further, since the sleeve member, which is externally fitted to the operation wire, is fixed to the member to be operated, other fixing members are not required. Accordingly, a fixing portion can be reduced in size.

## Description

### BACKGROUND OF THE INVENTION

### 1. Field of the Invention

The present invention relates to an operation mechanism, an endoscope, and a manufacturing method, and more particularly, to an operation mechanism that operates a member to be operated by an operation wire, an endoscope, and a manufacturing method.

### 2. Description of the Related Art

In an operation mechanism that operates a member to be operated by operation wires, the operation wires need to be fixed to the member to be operated. As disclosed in, for example, JP2016-34352A, in an endoscope that performs an operation for bending an insertion unit by pushing/pulling operation wires, the operation wires are inserted into wire guide pipes and are fitted to sleeves and the sleeves are fixed to a proximal end ring and a distal end ring.

### SUMMARY OF THE INVENTION

In JP2016-34352A, a part of (central sides of the proximal end ring and the distal end ring) of slits of the sleeves are notched to increase the internal space of the insertion unit. However, time is taken in the machining of the sleeves to form notches, and the wire guide pipes cannot be reliably fixed to the proximal end ring and the distal end ring since the openings of the slits are increased in size in a case in which the number of notches is too large. Further, since the operation wires are fitted to the wire guide pipes and are then fitted to the sleeves, a fixing portion is increased in size.

In the related art, the operation wires cannot be reliably fixed to the member to be operated as described above.

The invention has been made in consideration of the above-mentioned circumstances, and an object of the invention is to provide an operation mechanism, an endoscope, and a manufacturing method (a method of manufacturing the operation mechanism) that can reliably fix an operation wire to a member to be operated.

In order to achieve the above-mentioned object, an operation mechanism according to a first aspect of the invention comprises an operation wire that is formed by twisting a plurality of strands, a sleeve member that is externally fitted to the operation wire and is caulked against the operation wire in a caulked shape where a twisted shape of the plurality of strands is maintained, and a member to be operated to which the sleeve member is fixed. The member to be operated is operated according to an operation of the operation wire.

According to the first aspect, since the sleeve member is caulked in a caulked shape where a twisted shape of the plurality of strands of the operation wire is maintained, the sleeve member is caulked while the strands are maintained in an original line contact state. Accordingly, since damages to the strands caused by a point contact state of the strands do not occur, the tensile strength and bending durability of the operation wire are improved and the operation wire can be reliably fixed to the member to be operated. Further, since the sleeve member, which is externally fitted to the operation wire, is fixed to the member to be operated, other fixing members are not required. Accordingly, a fixing portion can be reduced in size.

In order to achieve the above-mentioned object, an operation mechanism according to a second aspect of the invention comprises an operation wire that is formed by twisting a plurality of strands, a sleeve member that is externally fitted to the operation wire and is caulked against the operation wire in a shape where a ratio of a diameter corresponding to a short side of the sleeve member to a diameter corresponding to a long side of the sleeve member in a plane orthogonal to a direction of a longitudinal axis of the operation wire is in the range of 0.6 to 1.0, and a member to be operated to which the sleeve member is fixed. The member to be operated is operated according to an operation of the operation wire. Since the caulked shape of the sleeve member is specifically specified in the second aspect, the tensile strength and bending durability of the operation wire are improved as in the first aspect by the caulking of the sleeve member in such a shape and the operation wire can be reliably fixed to the member to be operated. Further, since the sleeve member, which is externally fitted to the operation wire, is fixed to the member to be operated, other fixing members are not required. Accordingly, a fixing portion can be reduced in size. In a case in which a diameter in a horizontal direction in a plane orthogonal to the direction of the longitudinal axis of the operation wire is denoted by W and a diameter in a vertical direction orthogonal to the horizontal direction is denoted by H, "a ratio of a diameter corresponding to a short side of the sleeve member to a diameter corresponding to a long side of the sleeve member" (ellipticity) is expressed as a ratio between the diameters in the two directions orthogonal to each other, that is, a ratio (short side/long side) of "a diameter corresponding to a shorter side of a width W and a height H (short side)" to "a diameter corresponding to a longer side of the width W and the height H (long side)". In the second aspect, it is more preferable that the sleeve member is caulked in a shape where a ratio of the diameter corresponding to the short side to the diameter corresponding to the long side is in the range of 0.8 to 1.0.

According to an operation mechanism of a third aspect, in the second aspect, the sleeve member is fixed to the member to be operated so that a direction of the short side coincides with a radial direction of the member to be operated in the plane orthogonal to the direction of the longitudinal axis of the operation wire. In a case in which the sleeve member is fixed as in the third aspect, the fixing strength of the sleeve member can be increased in comparison with that in a case in which the sleeve member is fixed so that the direction of the long side of the sleeve member coincides with the radial direction of the member to be operated. Further, since the length of a portion of the sleeve member protruding into the member to be operated is reduced, the internal space of the member to be operated can be effectively used.

According to an operation mechanism of a fourth aspect, in any one of the first to third aspects, the member to be operated includes an opening portion and the sleeve member is fixed to the member to be operated at boundary portions where the sleeve member faces the opening portion. Since the member to be operated includes the opening portion in the fourth aspect, the sleeve member is easily positioned in a case in which the sleeve member is to be fixed and the operation wire can be reliably fixed to the member to be operated. Further, since the sleeve member is fixed at the boundary portions where the sleeve member faces the opening portion, a fixing portion can be reduced in size and height (by the thickness of the member to be operated).

According to an operation mechanism of a fifth aspect, in the fourth aspect, the sleeve member includes a projection portion and the sleeve member is fixed to the member to be operated in a state in which the projection portion is fitted to the opening portion. According to the fifth aspect, since the projection portion and the opening portion form a fitting structure, the sleeve member is easily positioned and the operation wire can be reliably fixed to the member to be operated.

According to an operation mechanism of a sixth aspect, in any one of the first to fifth aspects, the sleeve member and the member to be operated are fixed to each other by laser welding. A method of fixing operation wires to the inside of ring-shaped members by brazing, soldering, or the like is also considered in a case in which operation wires are to be fixed. However, since these ring-shaped members have a small diameter, it is difficult to fix the operation wires to the inside of the ring-shaped members. Further, since unnecessary "brazing filler metal" or "solder" protrudes, there is a case where the internal space of the ring-shaped member is narrowed. In contrast, according to the sixth aspect, since a reduction in the internal space of the member to be operated, which is caused by the protrusion of unnecessary "brazing filler metal" or "solder", does not occur and laser welding can be performed from the outside of the member to be operated, the operation wire can be easily fixed.

In order to achieve the above-mentioned object, an endoscope according to a seventh aspect comprises: the operation mechanism according to any one of the first to sixth aspects; an insertion unit that includes a hard distal end part, a bendable part including the member to be operated, and a flexible pipe part arranged in this order from a distal end thereof; and an operation unit that performs an operation for pushing/pulling the operation wire. The bendable part is bent according to the operation of the operation unit. Since the endoscope according to the seventh aspect comprises the operation mechanism according to any one of the first to sixth aspects, the operation wire is reliably fixed to the bendable part including the member to be operated.

In order to achieve the above-mentioned object, there is provided a method of manufacturing an operation mechanism according to an eighth aspect. The operation mechanism includes an operation wire that is formed by twisting a plurality of strands, a sleeve member that is caulked against the operation wire, and a member to be operated to which the sleeve member is fixed, and the member to be operated is operated according to an operation of the operation wire. The method comprises: a caulking step of caulking the sleeve member, which is externally fitted to the operation wire, in a caulked shape where a twisted shape of the plurality of strands is maintained; and a fixing step of fixing the sleeve member, which is caulked in the caulking step, to the member to be operated. According to the eighth aspect, since the sleeve member is caulked in a caulked shape where a twisted shape of the plurality of strands of the operation wire is maintained as in the first aspect, the sleeve member is caulked while the strands are maintained in an original line contact state. Accordingly, since damages to the strands caused by a point contact state of the strands do not occur, the tensile strength and bending durability of the operation wire are improved and the operation wire can be reliably fixed to the member to be operated. Further, since the sleeve member, which is externally fitted to the operation wire, is fixed to the member to be operated, other fixing members are not required. Accordingly, a fixing portion can be reduced in size.

In order to achieve the above-mentioned object, there is provided a method of manufacturing an operation mechanism according to a ninth aspect. The operation mechanism includes an operation wire that is formed by twisting a plurality of strands, a sleeve member that is caulked against the operation wire, and a member to be operated to which the sleeve member is fixed, and the member to be operated is operated according to an operation of the operation wire. The method comprises: a caulking step of caulking the sleeve member, which is externally fitted to the operation wire, in a shape where a ratio of a diameter corresponding to a short side of the sleeve member to a diameter corresponding to a long side of the sleeve member in a plane orthogonal to a direction of a longitudinal axis of the operation wire is in the range of 0.6 to 1.0; and a fixing step of fixing the sleeve member, which is caulked in the caulking step, to the member to be operated. Since the caulked shape of the sleeve member is specifically specified in the ninth aspect, the tensile strength and bending durability of the operation wire are improved as in the second aspect by the caulking of the sleeve member in such a shape and the operation wire can be reliably fixed to the member to be operated. Further, since the sleeve member, which is externally fitted to the operation wire, is fixed to the member to be operated, other fixing members are not required. Accordingly, a fixing portion can be reduced in size. In a case in which a diameter in a horizontal direction in a plane orthogonal to the direction of the longitudinal axis of the operation wire is denoted by W and a diameter in a vertical direction orthogonal to the horizontal direction is denoted by H, as in the second aspect, "a ratio of a diameter corresponding to a short side of the sleeve member to a diameter corresponding to a long side of the sleeve member" (ellipticity) is expressed as a ratio between the diameters in the two directions orthogonal to each other, that is, a ratio (short side/long side) of "a diameter corresponding to a shorter side of a width W and a height H (short side)" to "a diameter corresponding to a longer side of the width W and the height H (long side)". In the ninth aspect, it is more preferable that the sleeve member is caulked in a shape where a ratio of the diameter corresponding to the short side to the diameter corresponding to the long side is in the range of 0.8 to 1.0.

According to a manufacturing method of a tenth aspect, in the ninth aspect, the sleeve member is fixed to the member to be operated in the fixing step so that a direction of the short side coincides with a radial direction of the member to be operated in the plane orthogonal to the direction of the longitudinal axis of the operation wire. According to the tenth aspect, the fixing strength of the sleeve member can be increased as in the third aspect, and the internal space of the member to be operated can be effectively used since the length of a portion of the sleeve member protruding into the member to be operated is reduced.

According to a manufacturing method of an eleventh aspect, in any one of the eighth to tenth aspects, the member to be operated includes an opening portion and the sleeve member is fixed at boundary portions where the sleeve member faces the opening portion in the fixing step. According to the eleventh aspect, since the member to be operated includes an opening portion as in the fourth aspect, the sleeve member is easily positioned in a case in which the sleeve member is to be fixed and the operation wire can be reliably fixed to the member to be operated. Further, since the sleeve member is fixed at the boundary portions where the sleeve member faces the opening portion, a fixing portion can be reduced in size and height (by the thickness of the member to be operated).

According to a manufacturing method of a twelfth aspect, in the eleventh aspect, a projection portion is formed on the sleeve member in the caulking step and the sleeve member is fixed to the member to be operated in a state in which the projection portion is fitted to the opening portion in the fixing step. According to the twelfth aspect, since the projection portion and the opening portion form a fitting structure as in the fifth aspect, the sleeve member is easily positioned and the operation wire can be reliably fixed to the member to be operated.

According to a manufacturing method of a thirteenth aspect, in any one of the eighth to twelfth aspects, the sleeve member and the member to be operated are fixed to each other by laser welding in the fixing step. According to the thirteenth aspect, since a reduction in the internal space of the member to be operated, which is caused by the protrusion of unnecessary "brazing filler metal" or "solder", does not occur as in the sixth aspect and laser welding can be performed from the outside of the member to be operated, the operation wire can be easily fixed.

According to a manufacturing method of a fourteenth aspect, in the thirteenth aspect, the laser welding is performed in the fixing step so that an angle between a line connecting a laser emission position at the time of the laser welding with a center of the operation wire and a laser emission direction at the time of the laser welding is set to be larger than a half apex angle of the operation wire viewed from the laser emission position in a plane perpendicular to a longitudinal direction of the operation wire. In a case in which laser welding is to be performed, there is a concern that a laser emission direction may be inadequate and an operation wire may be damaged since the operation wire is present in a weld-penetration direction of the sleeve member. However, according to the fourteenth aspect, since the operation wire is not present in the weld-penetration direction, it is possible to reduce a concern that the operation wire may be damaged.

According to the operation mechanism, the endoscope, and the manufacturing method of the invention, as described above, the operation wire can be reliably fixed to the member to be operated.

### BRIEF DESCRIPTION OF THE DRAWINGS

Fig. 1 is a diagram showing the appearance of an endoscope to which the invention is applied.
Fig. 2 is a longitudinal sectional view of the periphery of a connecting portion between a bendable part and a hard distal end part taken along the longitudinal axis of an insertion unit.
Fig. 3 is a longitudinal sectional view of the periphery of a connecting portion between the bendable part and a flexible pipe part taken along the longitudinal axis of the insertion unit.
Fig. 4 is a diagram conceptually showing the bendable part and a bending operation mechanism.
Fig. 5 is a diagram showing an aspect in which operation wires are fixed to a distal end ring of the bendable part.
Fig. 6 is a diagram showing an aspect in which the distal end ring to which the operation wires are fixed is viewed from a distal end.
Fig. 7 is a diagram showing an aspect in which a sleeve is caulked.
Fig. 8 is a diagram illustrating the ellipticity of the sleeve.
Fig. 9 is a diagram showing an aspect in which the sleeve is fixed to the distal end ring by laser welding.
Fig. 10 is a diagram showing the distal end ring that is provided with opening portions.
Fig. 11 is a cross-sectional view showing the periphery of an opening portion of the distal end ring.
Fig. 12 is a diagram showing an aspect in which the sleeve is laser-welded to the distal end ring at a boundary portion facing the opening portion.
Fig. 13 is a diagram showing the direction of laser welding.
Fig. 14 is another diagram showing the direction of laser welding.
Fig. 15 is a diagram showing a state in which laser welding has been performed.
Fig. 16 is another diagram showing an aspect in which the sleeve is caulked.
Figs. 17A and 17B are diagrams showing a sleeve that is provided with a projection portion and an aspect in which the sleeve is fitted to the opening portion.
Fig. 18 is another diagram showing an aspect in which the sleeve is fixed to the distal end ring by laser welding.
Fig. 19 is another diagram showing the direction of laser welding.
Fig. 20 is a diagram showing an aspect in which a cross-section of a second embodiment is observed.
Fig. 21 is a graph showing a relationship between the shape and bending durability of a sleeve.
Fig. 22 is a graph showing a relationship between the shape and tensile strength of a sleeve.
Figs. 23A and 23B are diagrams showing an aspect in which a cross-section is observed in a case in which an ellipticity is 1.0.
Figs. 24A and 24B are diagrams showing an aspect in which a cross-section is observed in a case in which an ellipticity is 0.5.

### DESCRIPTION OF THE PREFERRED EMBODIMENTS

An operation mechanism, an endoscope, and a manufacturing method (a method of manufacturing the operation mechanism) according to embodiments of the invention will be described in detail below with reference to accompanying drawings.

### First embodiment

### Entire structure of endoscope

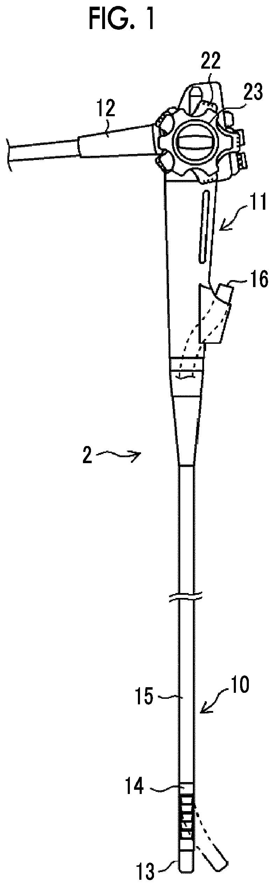

Fig. 1 is a diagram showing the appearance of an endoscope 2 (endoscope) to which the invention is applied. In Fig. 1, the endoscope 2 includes an insertion unit 10 (insertion unit) that is to be inserted into a body, an operation unit 11 (operation unit) that is connected to the proximal end portion of the insertion unit 10, and a universal cord 12 that is connected to the operation unit 11. The universal cord 12 is to connect the endoscope 2 to a processor device (not shown) that processes signals of an image taken by the endoscope 2 and a light source device (not shown) that emits illumination light to be guided to the endoscope 2, and is provided with a signal cable that is used to send image signals and control signals, a light guide that guides illumination light, and the like.

### Structure of insertion unit

The insertion unit 10 includes a hard distal end part 13 (hard distal end part), a bendable part 14 (bendable part), and a flexible pipe part 15 (flexible pipe part) that are arranged in this order from a distal end thereof. The bendable part 14 is connected to the proximal end of the hard distal end part 13, and the flexible pipe part 15 is connected to the proximal end of the bendable part 14. An imaging device that is used to image a portion to be observed in a body cavity, an illumination light emitting unit that irradiates the portion to be observed with illumination light, and the like are mounted in the hard distal end part 13. The flexible pipe part 15 has flexibility, and has a length of several meters to allow the hard distal end part 13 to reach a target position in the body. In a case in which an operator operates a vertical angle knob 22 (operation unit) and a lateral angle knob 23 (operation unit) of a bending operation mechanism provided in the operation unit 11, the bendable part 14 is vertically and laterally bent and changes the direction of the hard distal end part 13.

### Structure of bendable part

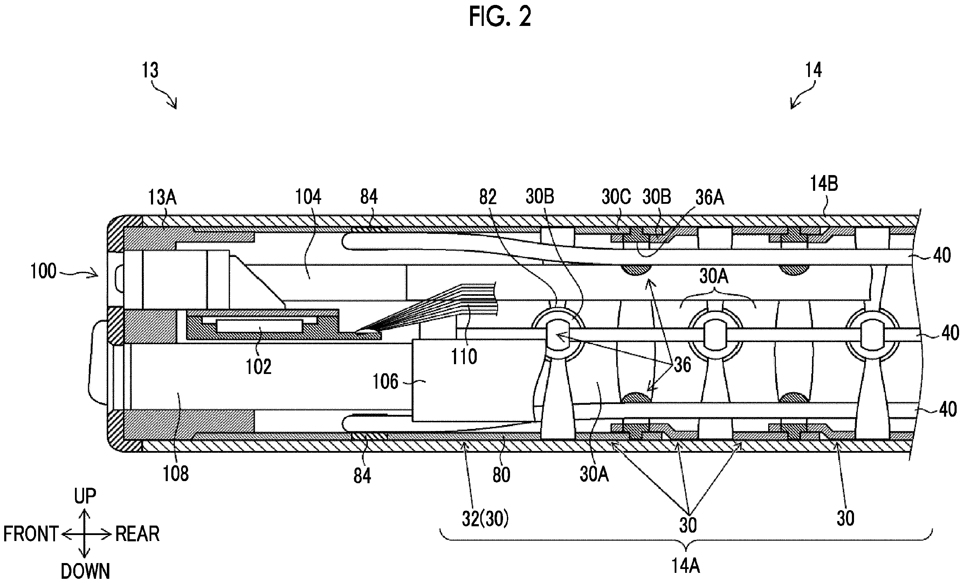

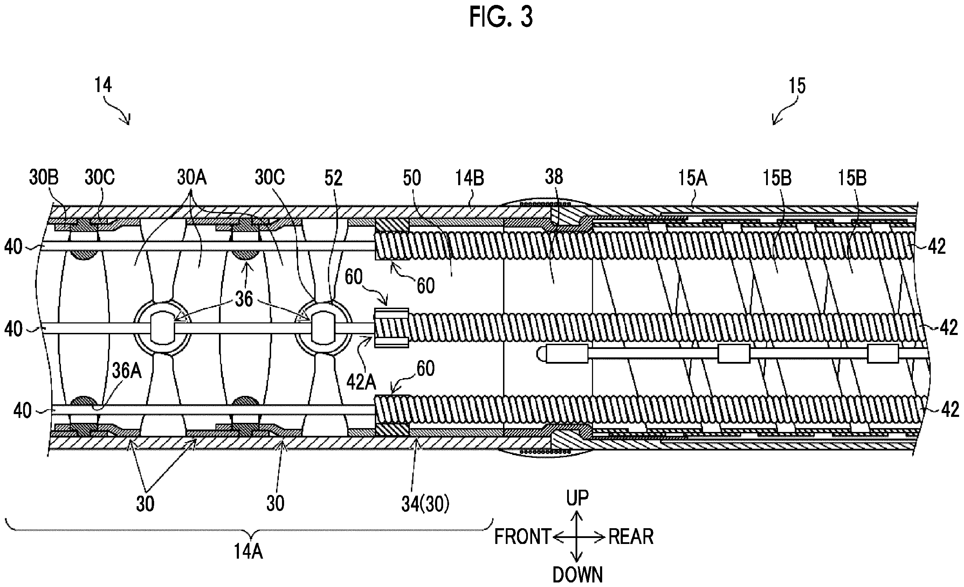

The structure of the bendable part 14 is shown in Figs. 2 and 3, Fig. 2 is a longitudinal sectional view of the periphery of a connecting portion between the bendable part 14 and the hard distal end part 13 taken along the longitudinal axis (axis) of the insertion unit, and Fig. 3 is a longitudinal sectional view of the periphery of a connecting portion between the bendable part 14 and the flexible pipe part 15 taken along the longitudinal axis of the insertion unit. As shown in Figs. 2 and 3, the bendable part 14 includes a nodal ring group 14A that is formed of a plurality of nodal rings 30 made of a metal material, such as a stainless steel material, and is adapted so that the outer peripheral side of the nodal ring group 14A is covered with an outer peripheral wall 14B including a mesh pipe and a covering.

The nodal rings 30 of the nodal ring group 14A are connected in series. Among these nodal rings 30, each of the nodal rings 30 excluding a distal end ring 32 (see Fig. 2; a member to be operated) that is a leading nodal ring 30 and a proximal end ring 34 (see Fig. 3) that is a tail nodal ring 30 includes a ring-shaped outer peripheral portion 30A, a pair of tongue pieces 30B and 30B that is formed so as to protrude on the distal end side of the outer peripheral portion 30A, and a pair of tongue pieces 30C and 30C that is formed so as to protrude on the proximal end side of the outer peripheral portion 30A.

The tongue pieces 30B and 30B provided on the distal end side and the tongue pieces 30C and 30C provided on the proximal end side are disposed on the nodal ring 30 at left and right positions and upper and lower positions that are different from each other in the circumferential direction. The tongue pieces 30C and 30C provided on the proximal end side of the nodal ring 30 of which the tongue pieces 30B and 30B provided on the distal end side are disposed at the left and right positions are disposed on the left and right sides, and the tongue pieces 30C and 30C provided on the proximal end side of the nodal ring 30 of which the tongue pieces 30B and 30B provided on the distal end side are disposed at the upper and lower positions are disposed on the upper and lower sides.

Further, the tongue pieces 30C and 30C provided on the proximal end side of a front nodal ring 30 of two nodal rings 30, which are adjacent to each other in a longitudinal direction, and the tongue pieces 30B and 30B provided on the distal end side of a rear nodal ring 30 thereof overlap each other at the upper and lower positions or the left and right positions and are connected to each other by connecting pins 36 so as to be rotationally movable. Accordingly, the nodal rings 30 are alternately connected to each other at the upper and lower positions and the left and right positions.

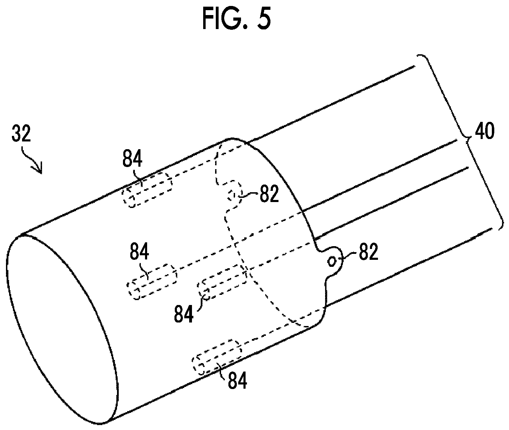

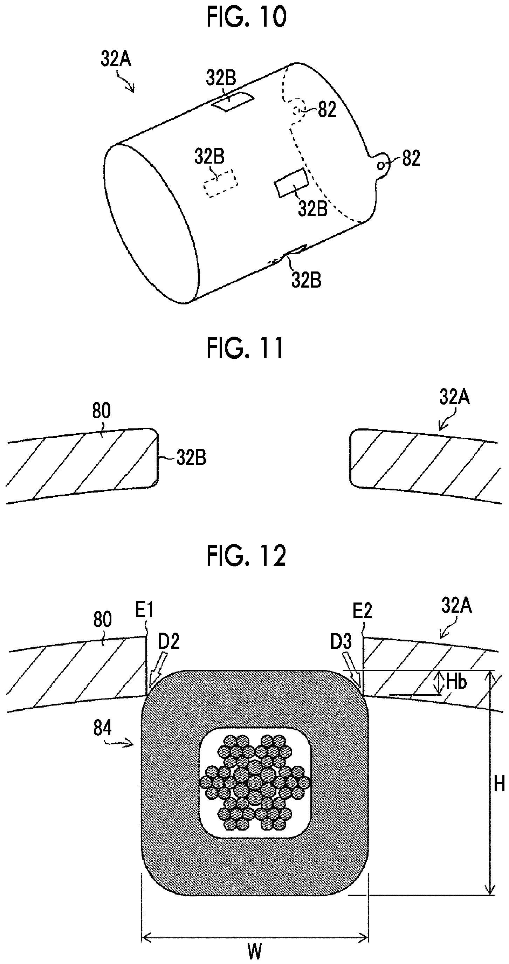

The distal end ring 32 that is the leading nodal ring 30 of the nodal ring group 14A includes a ring-shaped outer peripheral portion 80 as shown in Fig. 2, and tongue pieces 82 and 82 are provided on both left and right sides of the proximal end side of the outer peripheral portion 80 (see Figs. 5 and 10). Further, the tongue pieces 82 and 82 are connected to tongue pieces 30B and 30B provided on the distal end side of the rear nodal ring 30 next to the leading nodal ring 30 by the connecting pins 36 so as to be rotationally movable. Furthermore, the distal end side of the outer peripheral portion 80 is fixed to the proximal end of a body 13A of the hard distal end part 13.

The body 13A of the hard distal end part 13 is made of a metal material, such as a stainless steel material. An imaging optical system 100 and a solid-state imaging element 102 that form the imaging device used to image a portion to be observed, the illumination light emitting unit (not shown) that irradiates the portion to be observed with illumination light transmitted from the light source device through a light guide 104, a forceps pipe 108 to which a forceps tube 106 communicating with a forceps insertion part 16 of Fig. 1 is connected, and the like are assembled to the body 13A. A signal cable 110 that is connected to the imaging device of the hard distal end part 13, the light guide 104 that is connected to the illumination light emitting unit, the forceps tube 106 that is connected to the forceps pipe 108, an air/water supply tube, and the like are inserted into and disposed in the nodal rings 30 of the bendable part 14 and the flexible pipe part 15.

The proximal end ring 34 that is the tail nodal ring 30 of the nodal ring group 14A includes a ring-shaped outer peripheral portion 50 as shown in Fig. 3, and tongue pieces 52 and 52 are provided on both left and right sides of the distal end side of the outer peripheral portion 50. Further, the tongue pieces 52 and 52 are connected to tongue pieces 30C and 30C provided on the proximal end side of the front nodal ring 30 next to the tail nodal ring 30 by the connecting pins 36 so as to be rotationally movable. Furthermore, the proximal end side of the outer peripheral portion 50 is fixed to the distal end of a spiral pipe 15B, which is covered with a covering 15A, of the flexible pipe part 15 through an annular connecting ring 38.

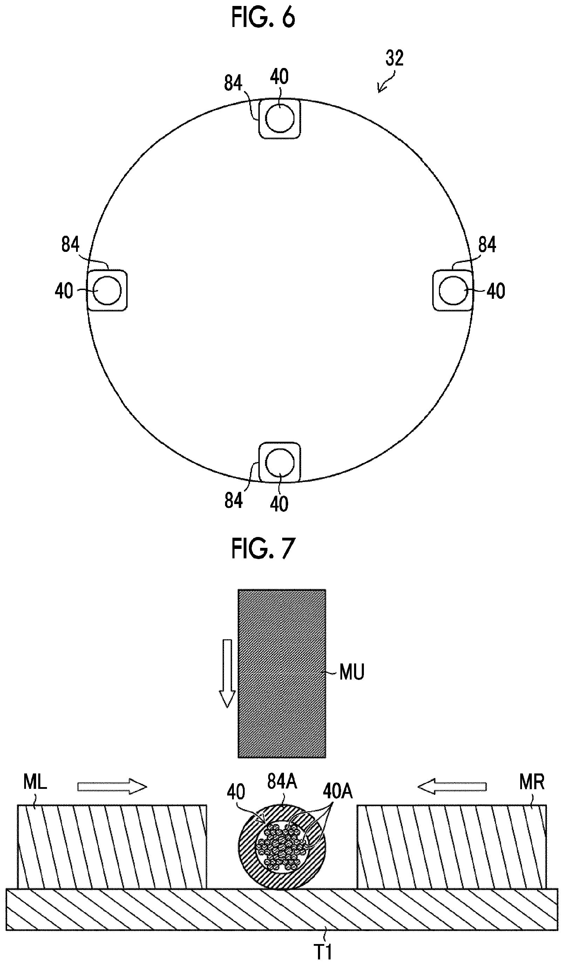

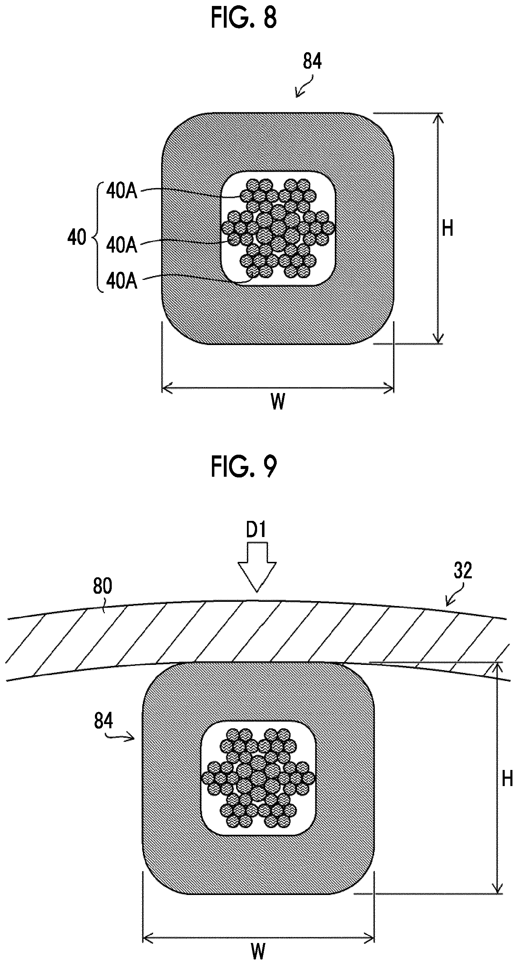

The bendable part 14 having the above-mentioned structure is vertically and laterally bent by an operation for pushing/pulling operation wires 40 (operation wires) that pass through the nodal rings 30 of the nodal ring group 14A. Four wires, which pass through the respective upper, lower, left, and right positions in the nodal rings 30, are provided as the operation wires 40, and the operation wires 40 are inserted into through-holes 36A of wire guide portions, which are formed integrally with the above-mentioned connecting pins 36 connecting the nodal rings 30 and protrude into the nodal rings 30, and are guided. As shown in Fig. 2, distal end portions of the operation wires 40 are held by sleeves 84 (sleeve members), which are provided on the outer peripheral portion 80 of the distal end ring 32 at the respective upper, lower, left, and right positions, so as to be fixed to the distal end ring 32. Further, the operation wires 40 are inserted into inner holes 42A of wire guide pipes 42 and are inserted into the flexible pipe part 15 as shown in Fig. 3, and proximal ends of the operation wires 40 are connected to the bending operation mechanism provided in the operation unit 11. A plurality of strands 40A are twisted together, so that each operation wire 40 is formed as one wire as a whole (see Figs. 7 and 8).

Each wire guide pipe 42 is formed of, for example, a closely wound coil and four wire guide pipes 42, which pass through the respective upper, lower, left, and right positions in the flexible pipe part 15, are provided so as to correspond to the four operation wires 40. As shown in Fig. 3, distal end portions of the wire guide pipes 42 are held by sleeves 60, which are provided on the outer peripheral portion 50 of the proximal end ring 34 at the respective upper, lower, left, and right positions, so as to be fixed to the proximal end ring 34. Proximal ends of the wire guide pipes 42 are fixed to the inside of the operation unit 11 or the proximal end portion of the flexible pipe part 15.

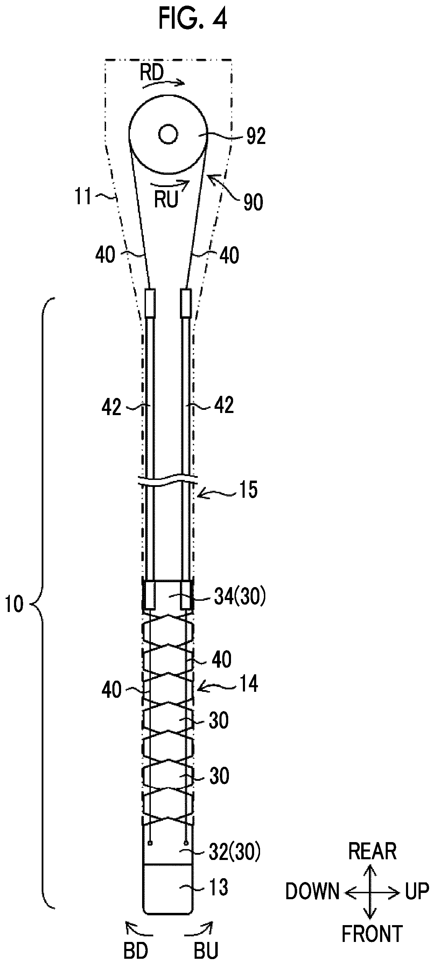

Fig. 4 is a diagram conceptually showing the bendable part 14 and the bending operation mechanism (one aspect of the operation mechanism) of the operation unit 11, and shows a structure that bends the bendable part 14 in a vertical direction (a vertical direction in the plane of paper). As shown in Fig. 4, in the bendable part 14, the plurality of nodal rings 30 are connected in series and adjacent nodal rings 30 are connected to each other so as to be relatively rotatable about an axis (the connecting pin 36) extending along a lateral direction (a direction perpendicular to the plane of paper). In regard to the nodal rings 30 of Fig. 4, two nodal rings, which are connected to each other so as to be relatively rotatable about an axis extending in the vertical direction (a vertical direction in the plane of paper) in Figs. 2 and 3, are shown as one nodal ring.

Further, among these nodal rings 30, the distal end ring 32, which is the leading nodal ring 30, is fixed to the hard distal end part 13 and the proximal end ring 34, which is the tail nodal ring 30, is fixed to the flexible pipe part 15. Accordingly, the bendable part 14 is adapted to be bendable in the vertical direction as a whole.

Further, the distal end portions of a pair of operation wires 40 and 40 are fixed to upper and lower portions of the inside of the distal end ring 32, respectively, and the distal end portions of a pair of wire guide pipes 42 and 42 into which the operation wires 40 are inserted are fixed to upper and lower portions of the inside of the proximal end ring 34, respectively.

The operation wires 40 are disposed so as to be inserted into the upper and lower portions in the nodal rings 30 and the wire guide pipes 42, and the proximal ends of the operation wires 40 are connected to the bending operation mechanism 90 (one aspect of the operation mechanism) provided in the operation unit 11. That is, the proximal ends of the operation wires 40 are wound on and fixed to a pulley 92 that forms the bending operation mechanism 90. According to this structure, the pulley 92 is rotated by an operator's operation for rotating the vertical angle knob 22 (see Fig. 1) of the operation unit 11, and the bendable part 14 is bent in an upward direction (the direction of an arrow BU in Fig. 4) in a case in which the pulley 92 is rotated in the direction of an arrow RU of Fig. 4. On the contrary, the bendable part 14 is bent in a downward direction (the direction of an arrow BD in Fig. 4) in a case in which the pulley 92 is rotated in the direction of an arrow RD of Fig. 4.

On the other hand, even in the case of the bending of the bendable part 14 in the lateral direction, likewise, the pair of operation wires 40 and the pair of wire guide pipes 42 are disposed at left and right portions in the bendable part 14 and the flexible pipe part 15 and the proximal ends of these operation wires 40 are wound on and fixed to a pulley (not shown) that is rotated in conjunction with an operation for rotating the lateral angle knob 23 (see Fig. 1) of the operation unit 11. Accordingly, the bendable part 14 is bent in the lateral direction by an operator's operation for rotating the lateral angle knob 23.

### Structure for fixing operation wire

Next, a structure for fixing the operation wires 40 will be described. As shown in Fig. 2, the operation wires 40 are fixed to the leading distal end ring 32 of the nodal ring group 14A of the bendable part 14 by the sleeves 84 (sleeve members).

Fig. 5 is a perspective view showing the distal end ring 32, the operation wires 40, and the sleeves 84 of the bendable part 14. Further, Fig. 6 is a diagram showing an aspect in which the distal end ring 32 to which the sleeves 84 are fixed is viewed from the distal end side. As shown in Figs. 5 and 6, the sleeves 84 are fixed to four portions, that is, upper, lower, left, and right portions of the inside of the outer peripheral portion 80 of the distal end ring 32 and the distal end portions of the operation wires 40 are held by these sleeves 84. Since all of the four sleeves 84, which are fixed to the outer peripheral portion 80 of the distal end ring 32, hold the distal end portions of the operation wires 40 by the same structure and method and are fixed to the outer peripheral portion 80, only the structure and the like of the sleeve 84 fixed to the upper portion of the inside of the outer peripheral portion 80 of the distal end ring 32 will be described below but the other sleeves 84 are also similar thereto.

### Caulking of sleeve

Fig. 7 is a cross-sectional view showing an aspect in which the sleeve is caulked. A sleeve 84A (sleeve member), which is not yet caulked, is a cylindrical member and is externally fitted to the distal end of the operation wire 40. Then, in a case in which the sleeve 84A is placed on a caulking table T1 and forces are applied to the sleeve 84A in the directions of arrows of Fig. 7 by a right die MR, a left die ML, and an upper die MU, a sleeve 84 (sleeve member) caulked so as to have a rectangular cross-section is obtained as shown in Fig. 8 (caulking step).

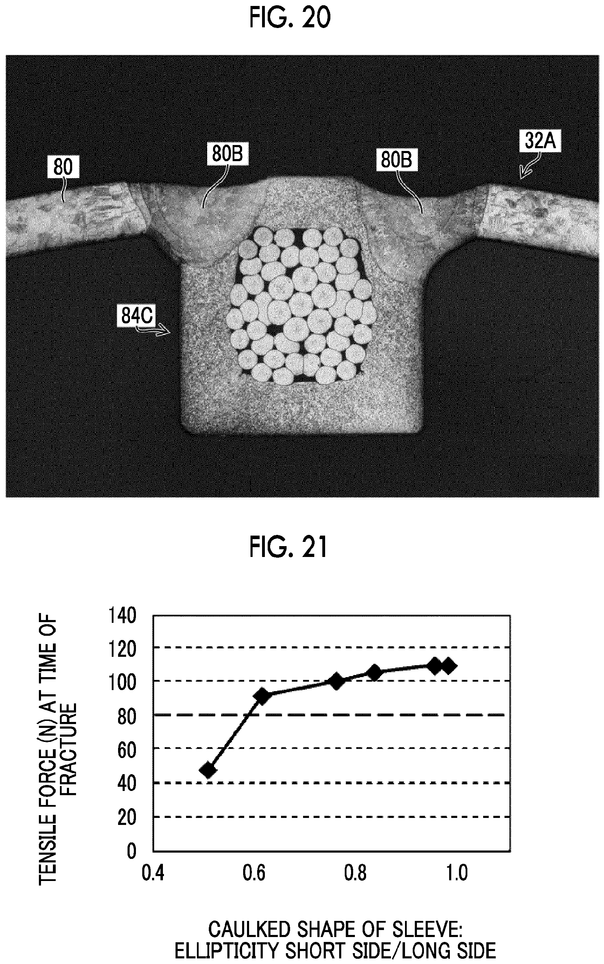

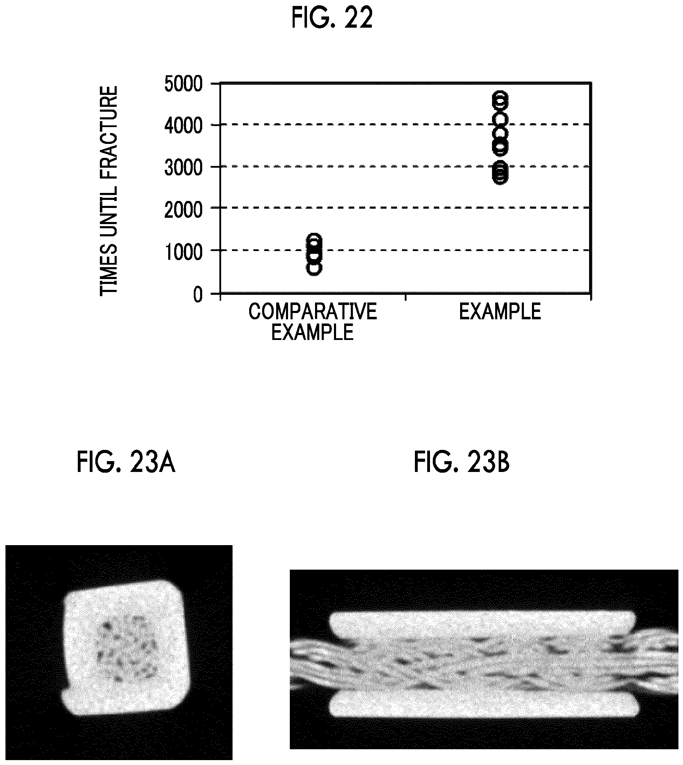

The shape of the caulked sleeve 84 is a shape where the twisted shape of the plurality of strands 40A (strands) forming the operation wire 40 is maintained. The maintenance of the twisted shape of the strands 40A (the maintenance of a line contact state) can be realized in a case in which the ellipticity of the caulked sleeve 84 is set in the range of 0.6 to 1.0, and the ellipticity of the sleeve 84 can be changed in a case in which forces applied to the right die MR, the left die ML, and the upper die MU and the like are changed. In a case in which a diameter in a horizontal direction in a plane orthogonal to the direction of the longitudinal axis of the operation wire 40 (in the plane of Fig. 8) is denoted by W and a diameter in a vertical direction orthogonal to the horizontal direction is denoted by H, an ellipticity is expressed as a ratio between the diameters in the two directions orthogonal to each other. That is, an ellipticity is expressed as a ratio (short side/long side) of "a diameter corresponding to a shorter side of a width W and a height H (short side)" to "a diameter corresponding to a longer side of the width W and the height H (long side)". In a case in which the ellipticity of the sleeve 84 is set to such a value, the sleeve 84 is caulked while the twisted shape of the strands 40A of the operation wire 40 is maintained and the strands 40A are maintained in an original line contact state. Accordingly, since damages to the strands 40A caused by the point contact state of the strands 40A do not occur, the tensile strength and bending durability of the operation wire 40 are improved and the operation wire 40 can be reliably fixed to the distal end ring 32 (see Figs. 21 and 22).

In a case in which the sleeve 84 caulked with such an ellipticity is to be fixed to the distal end ring 32, it is preferable to fix the sleeve 84 to the distal end ring so that the direction of the short side of the sleeve 84 coincides with the radial direction of the distal end ring 32. In a case in which the sleeves 84 are fixed in such a direction, the fixing strength of the sleeve can be increased in comparison with that in a case in which the sleeves 84 are fixed so that the direction of the long side of each sleeve 84 coincides with the radial direction of the distal end ring 32. Further, since the length of a portion of each sleeve 84 protruding into the distal end ring 32 is reduced, the internal space of the distal end ring 32 can be effectively used.

### Fixing of sleeve

The sleeve 84, which has been caulked in the above-mentioned shape, can be fixed to the distal end ring 32 by laser welding. According to laser welding shown in the following examples 1 and 2, since unnecessary "brazing filler metal" or "solder" does not protrude unlike in the case of fixing using brazing or soldering, the internal space of the distal end ring 32 is not narrowed.

### Example 1

In Example 1, laser is emitted in a laser emission direction D1 in a state in which each sleeve 84 is disposed on the inside of the outer peripheral portion 80 of the distal end ring 32 as shown in Fig. 9, so that the sleeve 84 is fixed (fixing step). The fixing using laser welding is performed along the length direction of the distal end ring 32. However, the fixing does not necessarily need to be continuously performed along the length direction, and may be performed using welding at one point in a case in which strength is ensured. Since the operation wire 40 is present in the laser emission direction D1 as shown in Fig. 9 in Example 1, it is preferable to adjust the output of laser so that the operation wire 40 is not damaged by the weld penetration of the distal end ring 32 and the sleeve 84.

### Example 2

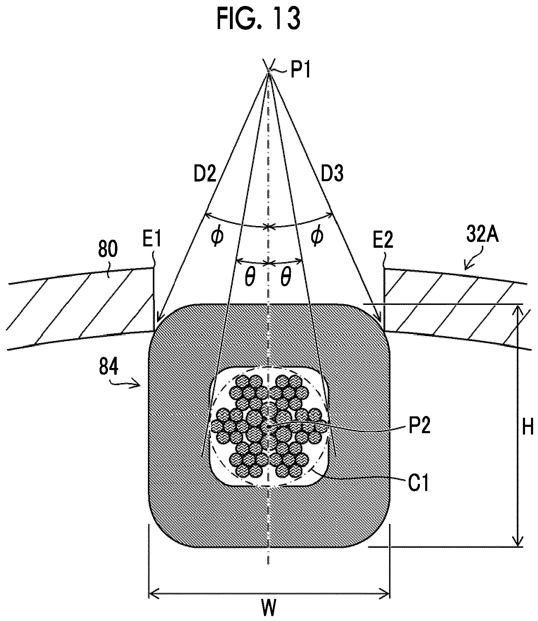

In Example 2, a distal end ring 32A (a member to be operated) is provided with opening portions 32B (opening portions) as shown in Figs. 10 and 11 and the sleeve 84 is positioned at each opening portion 32B (see Figs. 10 and 11) as shown in Fig. 12 (fixing step). The width of the opening portion 32B is set to be equal to or smaller than the width (W) of the sleeve 84. Accordingly, since the protrusion of the sleeve 84 into the distal end ring 32A is reduced (by Hb of Fig. 12), a fixing portion can be reduced in size. Then, laser is emitted in laser emission directions D2 and D3 of Fig. 12 to weld the sleeve 84 at boundary portions E1 and E2 of each opening portion 32B (portions where the sleeve 84 faces the opening portion 32B) (fixing step). The fixing using laser welding is performed along the length direction of the opening portion 32B (the longitudinal direction of the distal end ring 32A). As shown in Fig. 13, laser welding is performed so that an angle ϕ between a line connecting a laser emission position P1 with a center P2 of an imaginary circumscribed circle C1 of the operation wire 40 and each of the laser emission directions D2 and D3 is set to be larger than a half apex angle θ of the circumscribed circle C1 of the operation wire 40 viewed from the laser emission position P1 in a plane perpendicular to the longitudinal direction of the operation wire 40. The circumscribed circle C1 means the imaginary outer diameter of the operation wire 40. Accordingly, it is possible to avoid damage to the operation wire 40 that is caused by the weld penetration of the distal end ring 32 and the sleeve 84.

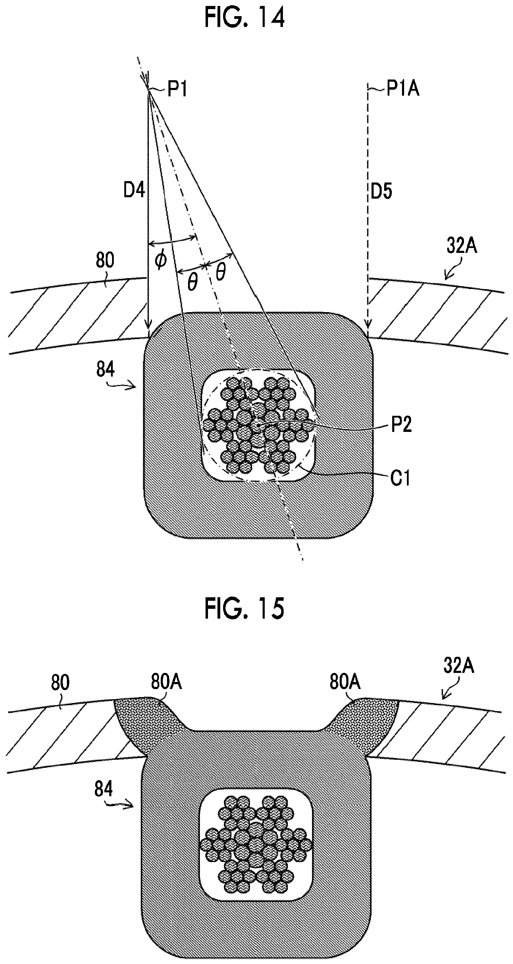

The laser emission position P1 can coincide with the middle of the width W as shown in Fig. 13, but may not coincide with the middle of the width W as shown in Fig. 14. In the case of Fig. 14, laser is emitted along the longitudinal direction of the opening portion 32B with respect to a laser emission direction D4, and laser is emitted from a laser emission position P1A in a laser emission direction D5 after the laser emission position and a work (a work holding the distal end ring and the sleeves 84) are moved relative to each other. In the aspect of Fig. 14, laser welding can be performed with only the movement of a machining device in the horizontal direction in a case in which the machining device cannot change the laser emission position and the angle of the work.

Regions 80A at which the boundary portions E1 and E2 are welded and penetrated are formed as shown in Fig. 15 after laser welding.

According to the operation mechanism (the operation wires 40, the sleeves 84, and the distal end ring 32 or 32A) of the first embodiment and the endoscope 2 including the operation mechanism, as described above, the operation wires 40 can be reliably fixed to the distal end ring 32 or 32A and the internal space of the distal end ring 32 or 32A can be increased in size by a reduction in the size of a fixing portion.

### Second embodiment

Next, an operation mechanism, an endoscope, and a manufacturing method (a method of manufacturing the operation mechanism) according to a second embodiment will be described. In the above-mentioned first embodiment, each sleeve 84 has been caulked in a rectangular shape (see Fig. 8 and the like). However, in the second embodiment, a projection portion is formed on each sleeve by caulking and laser welding is performed in a state in which the projection portion is fitted to each opening portion provided at a distal end ring 32. Since the structure of each portion of the endoscope is the same as that of the first embodiment, the detailed description thereof will be omitted with reference to Figs. 1 to 4.

### Caulking of sleeve

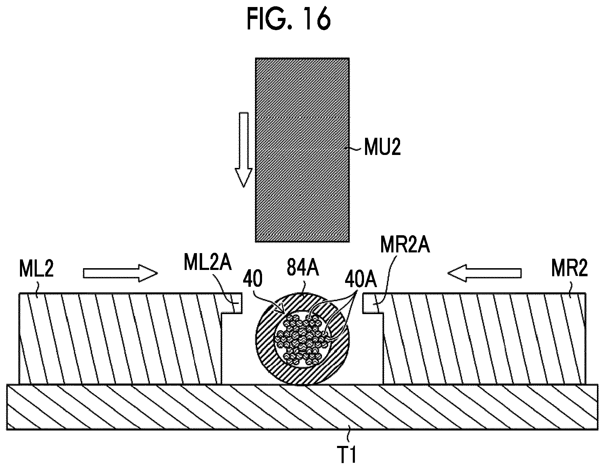

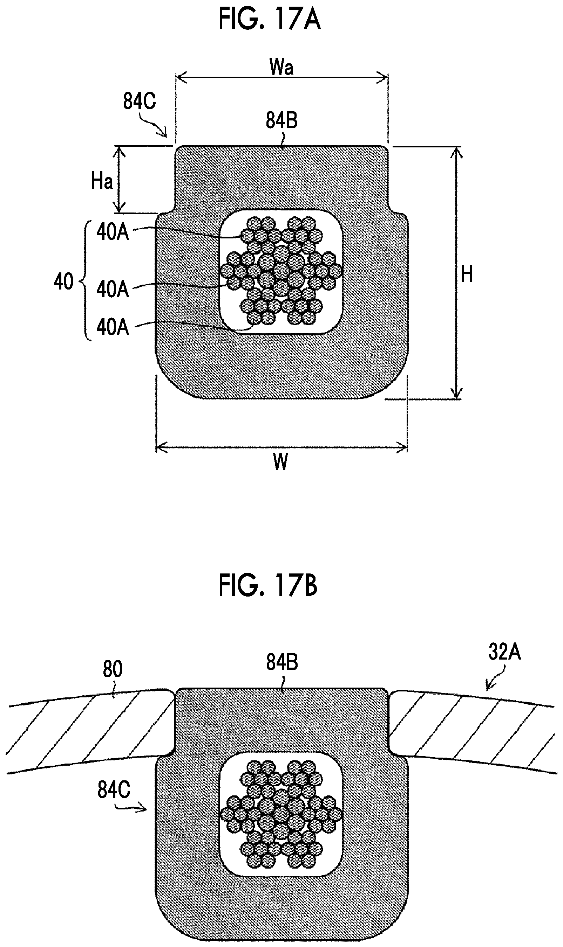

Fig. 16 is a cross-sectional view showing an aspect in which the sleeve is caulked in the second embodiment. A sleeve 84A (sleeve member), which is not yet caulked, is a cylindrical member and is externally fitted to the distal end of the operation wire 40. As in the first embodiment, the operation wire 40 is formed by twisting a plurality of strands 40A (strands). Then, in a case in which the sleeve 84A is placed on a caulking table T1 and forces are applied to the sleeve 84A in the directions of arrows of Fig. 16 by a right die MR2, a left die ML2, and an upper die MU2, a sleeve 84C (sleeve member) at which a projection portion 84B (projection portion) having a width Wa is formed is obtained as shown in Fig. 17A (caulking step). As shown in Fig. 16, projections MR2A and ML2A corresponding to the height of the projection portion 84B (a height Ha in Fig. 17A) are formed on the right and left dies MR2 and ML2, respectively.

Even in the case of the sleeve 84C caulked as shown in Figs. 17A and 17B, as in the first embodiment, an ellipticity (short side/long side) can be defined, and the sleeve 84C can be reliably fixed to the distal end ring 32A (a member to be operated) and tensile strength and bending durability can be improved (see Figs. 21 and 22) in a case in which the ellipticity is set in the range of 0.6 to 1.0, as described later. The projection portion 84B is formed on the long side of the sleeve 84C. Accordingly, in a case in which the projection portion 84B is to be fitted to the opening portion 32B, it is preferable to fix the sleeve 84C so that the direction of the short side of the sleeve 84C coincides with the radial direction of the distal end ring 32A. Specifically, it is preferable to form the projection portion 84B on the side having the width W (the upper side of the sleeve 84C in the example of Figs. 17A and 17B) so that the width W is set to be equal to or larger than the height H, and it is preferable to fix the sleeve 84C so that the direction of the height H (the direction of the short side) coincides with the radial direction of the distal end ring 32A. Accordingly, the fixing strength of the sleeve can be increased, and the internal space of the distal end ring 32A can be effectively used since the length of a portion of each sleeve 84C protruding into the distal end ring 32A is reduced.

### Fixing of sleeve

After the sleeves 84C are obtained through the above-mentioned caulking, the projection portion 84B is fitted to each opening portion 32B (see Fig. 11) of the distal end ring 32A as shown in Fig. 17B (fixing step). According to the fitting structure between the projection portion 84B and the opening portion 32B, a fixing portion can be reduced in size by the height Ha and the positioning of the sleeve is easy at the time of fixing. A clearance (for example, about 20 µm) may be formed between the projection portion 84B and the opening portion 32B so that fitting is easily performed.

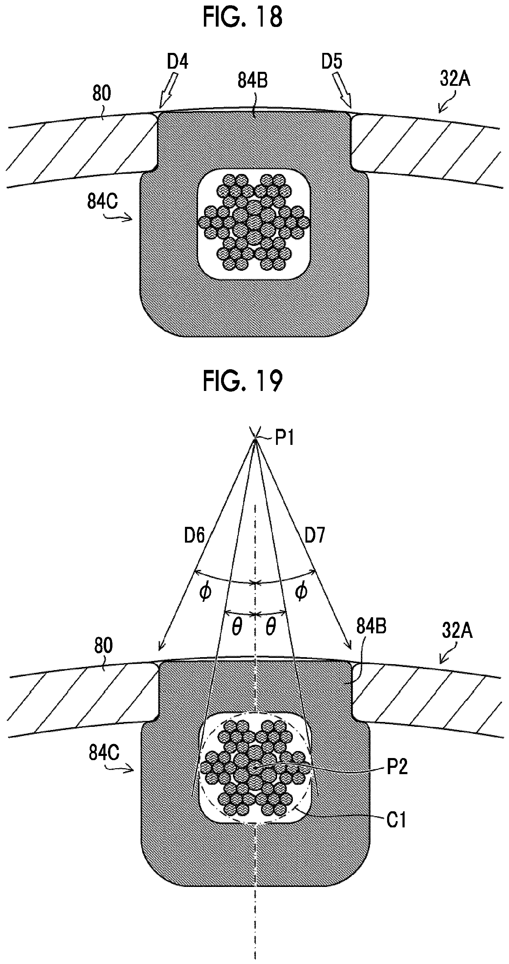

After the projection portion 84B is fitted to each opening portion 32B, laser is emitted in laser emission directions D4 and D5 of Fig. 18 to weld the sleeve 84 at boundary portions E1 and E2 of each opening portion 32B (fixing step). The fixing using laser welding is performed along the length direction of the opening portion 32B (the longitudinal direction of the distal end ring 32A). However, the fixing does not necessarily need to be continuously performed along the length direction, and may be performed using welding at one point in a case in which strength is ensured. In the laser welding, as shown in Fig. 19, an angle ϕ between a line connecting a laser emission position P1 with a center P2 of an imaginary circumscribed circle C1 of the operation wire 40 and each of laser emission directions D6 and D7 is set to be larger than a half apex angle θ of the circumscribed circle C1 of the operation wire 40 viewed from the laser emission position P1 in a plane perpendicular to the longitudinal direction of the operation wire 40. As in the case of Fig. 13, the circumscribed circle C1 means the imaginary outer diameter of the operation wire 40. It is possible to avoid damage to the operation wire 40, which is caused by the weld penetration of the distal end ring 32A and the sleeve 84C, by performing laser welding at such an angle. The laser emission position PI can coincide with the middle of the width W as shown in Fig. 19, but may coincide with not the middle of the width W but an end portion of the width W as shown above in Fig. 14. Fig. 20 is a diagram showing an aspect in which a cross-section of the second embodiment is observed after laser welding (an ellipticity is 0.94). Regions 80B at which the boundary portions of the opening portion 32B are welded and penetrated are formed as in Fig. 15, but the twisted shape of the strand 40A is maintained and the operation wire 40 is not damaged by laser welding.

According to the operation mechanism (the operation wires 40, the sleeves 84C, and the distal end ring 32A) of the second embodiment and the endoscope 2 including the operation mechanism, as described above, the operation wires 40 can be reliably fixed to the distal end ring 32A and the internal space of the distal end ring 32A can be increased in size by a reduction in the size of a fixing portion.

### Relationship between caulked shape and tensile strength

A relationship between the caulked shape (ellipticity; short side/long side) of the sleeve described in the first and second embodiments and tensile strength is shown in Fig. 21. As shown in Fig. 21, in order to make a tensile force at the time of fracture high (here, 80 N or more), it is preferable that an ellipticity is set in the range of 0.6 to 1.0 and it is more preferable that an ellipticity is set in the range of 0.8 to 1.0.

### Relationship between caulked shape and bending durability

A relationship between the caulked shape (ellipticity) of the sleeve described in the first and second embodiments and bending durability is shown in Fig. 22 (a tensile force is set to 50 N and a swinging angle is set in the range of 0° to 20°). In Fig. 22, an ellipticity is 1.0 in an example and an ellipticity is 0.5 in a comparative example. As shown in Fig. 22, in order to make bending durability high, it is preferable that an ellipticity is set in the range of 0.6 to 1.0.

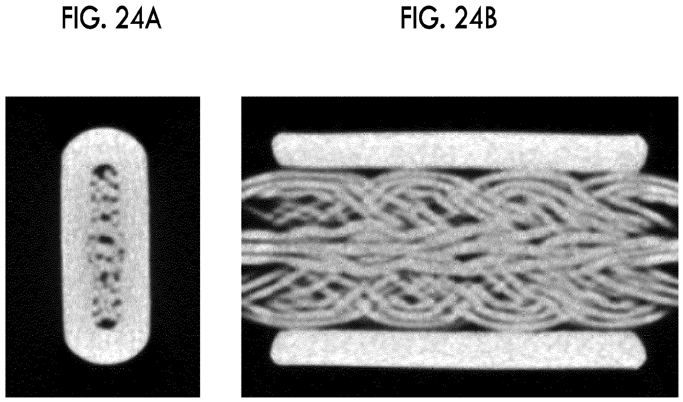

Figs. 23A and 23B are diagrams showing an aspect in which a cross-section is observed using X-ray computed tomography in a non-destructive manner in a case in which an ellipticity is in the range of 0.6 to 1.0, and Figs. 24A and 24B are diagrams showing an aspect in which a cross-section is observed using X-ray computed tomography in a non-destructive manner in a case in which an ellipticity is not in the range of 0.6 to 1.0. Figs. 23A and 23B correspond to a case in which an ellipticity is 1.0, and Fig. 23A shows a cross-section in a plane perpendicular to the longitudinal axis of the operation wire 40. Fig. 23B shows a cross-section in a plane parallel to the longitudinal axis of the operation wire. In a case in which an ellipticity is 1.0 (in a case in which an ellipticity is in the range of 0.6 to 1.0), it is understood that the twisted shape of the strands 40A of the operation wire 40 is maintained as shown in Figs. 23A and 23B. On the other hand, Figs. 24A and 24B correspond to a case in which an ellipticity is 0.5, and Fig. 24A shows a cross-section in a plane perpendicular to the longitudinal axis of the operation wire 40. Fig. 24B shows a cross-section in a plane parallel to the longitudinal axis of the operation wire. In a case in which an ellipticity is 0.5 (in a case in which an ellipticity is not in the range of 0.6 to 1.0), it is understood that the twisted shape of the strands 40A of the operation wire 40 is deformed as shown in Figs. 24A and 24B.

The embodiments of the invention have been described above. However, the invention is not limited to the above-mentioned aspects, and may have various modifications without departing from the scope of the invention. For example, the sleeves have been caulked in a rectangular shape and a rectangular shape including a projection portion in the above-mentioned first and second embodiments, but the sleeve may be caulked in a circular shape, an oval shape, or the like. Further, the invention can be applied to overall devices, to which mechanical operations, such as a push, a pull, and rotation, can be applied, other than an operation mechanism for an endoscope. For example, the invention can be applied to a mechanism for opening/closing a window, blinds, or the like, or an automotive control cable for a trunk opener, a door lock, a transmission, a brake, or the like.

### Explanation of References

2: endoscope
10: insertion unit
11: operation unit
12: universal cord
13: hard distal end part
13A: body
14: bendable part
14A: nodal ring group
14B: outer peripheral wall
15: flexible pipe part
15A: covering
15B: spiral pipe
16: forceps insertion part
22: vertical angle knob
23: lateral angle knob
30: nodal ring
30A: outer peripheral portion
30B: tongue piece
30C: tongue piece
32: distal end ring
32A: distal end ring
32B: opening portion
34: proximal end ring
36: connecting pin
36A: through-hole
38: connecting ring
40: operation wire
40A: strand
42: wire guide pipe
42A: inner hole
50: outer peripheral portion
52: tongue piece
60: sleeve
80: outer peripheral portion
80A: region
80B: region
82: tongue piece
84: sleeve
84A: sleeve
84B: projection portion
84C: sleeve
90: bending operation mechanism
92: pulley
100: imaging optical system
102: solid-state imaging element
104: light guide
106: forceps tube
108: forceps pipe
110: signal cable
BD: arrow
BU: arrow
C1: circumscribed circle
D1: laser emission direction
D2: laser emission direction
D3: laser emission direction
D4: laser emission direction
D5: laser emission direction
D6: laser emission direction
D7: laser emission direction
E1: boundary portion
E2: boundary portion
H: height
Ha: height
ML: left die
ML2: left die
MR: right die
MR2: right die
MR2A: projection
MU: upper die
MU2: upper die
P1: laser emission position
P2: center
RD: arrow
RU: arrow
T1: caulking table
W: width
Wa: width

## Claims

1. An operation mechanism comprising:
an operation wire that is formed by twisting a plurality of strands;
a sleeve member that is externally fitted to the operation wire and is caulked against the operation wire in a shape where a ratio of a diameter corresponding to a short side of the sleeve member to a diameter corresponding to a long side of the sleeve member in a plane orthogonal to a direction of a longitudinal axis of the operation wire is in the range of 0.6 to 1.0; and
a member to be operated to which the sleeve member is fixed,
wherein the member to be operated is operated according to an operation of the operation wire.

2. The operation mechanism according to claim 1,
wherein the sleeve member is externally fitted to the operation wire and is caulked against the operation wire in a caulked shape where a twisted shape of the plurality of strands is maintained.

3. The operation mechanism according to claim 1 or 2,
wherein the sleeve member is fixed to the member to be operated so that a direction of the short side coincides with a radial direction of the member to be operated in the plane orthogonal to the direction of the longitudinal axis of the operation wire.

4. The operation mechanism according to any one of claims 1 to 3,
wherein the member to be operated includes an opening portion, and
the sleeve member is fixed to the member to be operated at boundary portions where the sleeve member faces the opening portion.

5. The operation mechanism according to claim 4,
wherein the sleeve member includes a projection portion, and
the sleeve member is fixed to the member to be operated in a state in which the projection portion is fitted to the opening portion.

6. The operation mechanism according to any one of claims 1 to 5,
wherein the sleeve member and the member to be operated are fixed to each other by laser welding.

7. An endoscope comprising:
the operation mechanism according to any one of claims 1 to 6;
an insertion unit that includes a hard distal end part, a bendable part including the member to be operated, and a flexible pipe part arranged in this order from a distal end thereof; and
an operation unit that performs an operation for pushing/pulling the operation wire,
wherein the bendable part is bent according to the operation of the operation unit.

8. A method of manufacturing an operation mechanism, the operation mechanism including an operation wire that is formed by twisting a plurality of strands, a sleeve member that is caulked against the operation wire, and a member to be operated to which the sleeve member is fixed, and the member to be operated being operated according to an operation of the operation wire, the method comprising:
a caulking step of caulking the sleeve member, which is externally fitted to the operation wire, in a shape where a ratio of a diameter corresponding to a short side of the sleeve member to a diameter corresponding to a long side of the sleeve member in a plane orthogonal to a direction of a longitudinal axis of the operation wire is in the range of 0.6 to 1.0; and
a fixing step of fixing the sleeve member, which is caulked in the caulking step, to the member to be operated.

9. The method according to claim 8,
wherein in the caulking step, the sleeve member is externally fitted to the operation wire and caulked in a caulked shape where a twisted shape of the plurality of strands is maintained.

10. The method according to claim 8 or 9,
wherein the sleeve member is fixed to the member to be operated in the fixing step so that a direction of the short side coincides with a radial direction of the member to be operated in the plane orthogonal to the direction of the longitudinal axis of the operation wire.

11. The method according to any one of claims 8 to 10,
wherein the member to be operated includes an opening portion, and
the sleeve member is fixed at boundary portions where the sleeve member faces the opening portion in the fixing step.

12. The method according to claim 11,
wherein a projection portion is formed on the sleeve member in the caulking step, and
the sleeve member is fixed to the member to be operated in a state in which the projection portion is fitted to the opening portion, in the fixing step.

13. The method according to any one of claims 8 to 12,
wherein the sleeve member and the member to be operated are fixed to each other by laser welding in the fixing step.

14. The method according to claim 13,
wherein the laser welding is performed in the fixing step so that an angle between a line connecting a laser emission position at the time of the laser welding with a center of the operation wire and a laser emission direction at the time of the laser welding is set to be larger than a half apex angle of the operation wire viewed from the laser emission position in a plane perpendicular to a longitudinal direction of the operation wire.
